# EUROPÄISCHE PATENTANMELDUNG

(11) **EP 2 078 514 A1**
(43) Veröffentlichungstag der Anmeldung: **15.07.2009**
(21) Anmeldenummer: 09150124.7
(22) Anmeldetag: 06.01.2009
(51) Int. Cl.: A61F 5/445

(54) **Stoma-Schutzkappe**

(30) Priorität: 11.01.2008 DE 102008004174
(71) Anmelder: Redlich, Hartmut, 69427 Mudau (DE)
(72) Erfinder: Redlich, Hartmut, 69427 Mudau (DE)
(74) Vertreter: Kewitz, Ansgar

(57) **Zusammenfassung**

Die Stoma- Schutzkappe zum Schutz der Stoma, umfassend eine Platte (4), die eine Wölbung (3) ausweist, wobei die Wölbung (3) so ausgebildet ist, dass sie einen Hohlraum über die Stoma aufspannt und die Platte mindestens einen Haltebereich umfasst (1), der eine Befestigung an einer Kleidung erlaubt, so dass die Platte zwischen der Kleidung und dem Körper sicher haltbar ist.

## Beschreibung

Die Erfindung betrifft eine Schutzkappe für einen Stoma bzw. eines künstlichen Darmausgangs.

### Gebiet der Erfindung

Zur medizinischen Versorgung nach Anlage eines Darmausganges (Kolostomie/Jleostomie) wird an den Stoma eine Basisplatte angelegt, welche durch einen Schließring mit einem Beutel verbunden wird, der für die Aufnahme des Stuhles dient.
Es versteht sich, dass die Erfindung nicht auf den Darmausgang beschränkt ist, sondern ebenfalls für die Urostomi-Versorgung angewendet werden kann. Bei dieser handelt es sich um einen künstlichen Blasenausgang der in gleicher Weise wie beim Stoma mit Basisplatte und einem Beutel ausgestattet ist. Im Folgenden ist der Begriff Stoma sowohl für beide Anwendungsfälle zu verstehen und nicht beschränkt zu betrachten.

Diese medizinische Versorgung besteht im Wesentlichen aus einer Basisplatte und einem Auffangbeutel für den Stuhl, die mit einem Schließringsystem verbunden werden. Dieses Schließringsystem besteht aus einem flexiblen Klebepflaster, das mit einem stabileren Schließring versehen ist, auf den der Auffangbeutel geklemmt wird.
Um dieses System vor Druck zu schützen, gibt es im sanitären Angebot einen Schutz in Verbindung mit einem Bruchband, wobei der Stomabeutel durch ein Loch in dem Bruchbandes gezogen wird und durch eine Plastikkappe, welche durch einen Klettstreifen über dem Loch des Bruchbandes befestigt ist, gegen Druck geschützt ist. In diesem Zusammenhang wird auf die Druckschriften (DE 299 00 295 U1, DE 1786 120, DE 1714036U, DD 39645A1,DE verwiesen.
Dieser Ansatz bringt jedoch eine Reihe von Problemen mit sich, wie z. B. das Zusammendrücken des Beutels.

### Überblick über die Erfindung:

Aufgabe der Erfindung ist eine einfachere Schutzkappe bereitzustellen, die einen deutlich besseren Schutz bietet.
Gelöst wird diese Aufgabe durch eine Anordnung bzw. Vorrichtung mit den Merkmalen des unabhängigen Anspruchs. Mit der neuen Stoma- Schutzkappe werden wesentliche Probleme gelöst, wie sie weiter unten beschrieben werden.

Hierbei wird eine Kappe verwendet, die vorzugsweise auf den Hosenbund oder einen Gürtel geklemmt wird. Im Einzelnen handelt es sich bei der Stoma- Schutzkappe, um eine stabile hautfreundliche Platte. Die Platte weist eine Wölbung auf, die so ausgebildet ist, dass sie einen Hohlraum über den Stoma aufspannt. Hierdurch soll ein direkter Kontakt der Kleidung bzw. des Gürtels mit dem Stoma vermieden werden. Damit die Platte nicht verrutscht, umfasst diese mindestens einen Haltebereich, der eine Befestigung an der Kleidung erlaubt, so dass die Platte zwischen der Kleidung und dem Körper sicher gehalten wird. Der Haltebereich kann unterschiedlicher Art sein. So kann an einen Klemmbereich, Einhakbereiche an Druckknopfbereiche, an Klettbereiche oder ähnliches gedacht werden.

In der bevorzugten Ausführungsform ist ein Klemmbereich oder ein Einhakbereich gegeben, der es erlaubt die Stoma-Schutzkappe in die Kleidung einzuhaken. Das Einhaken erfolgt vorzugsweise auf den Bund der Hose oder hinter einen Gürtel.

Der Einhakbereich, der auch als Führungen bezeichnet wird, ist in einer bevorzugten Form langlochförmig ausgebildet. Wobei die langlochförmigen Führungen von unten nach oben verlaufen (hierbei wird die Platte in der verwendeten Form betrachtet). Der untere Bereich der Führung ist geöffnet ausgebildet, wobei der obere Bereich des Langlochs in der Stoma- Schutzkappe endet, so dass ein Aufschieben auf einen Hosenbund oder einen Gürtel von oben nach unten ermöglicht wird. Der Hosenbund bzw. der Gürtel werden hierbei in die langlochförmigen Führungen eingefädelt. Betrachtet man den die Stoma- Schutzkappe von links nach rechts, so wird der Hosenbund oder der Gürtel im ersten Bereich unterhalb der Stoma- Schutzkappe geführt, dann wird er durch das Langloch vor der Stoma- Schutzkappe über die Wölbung geführt, um dann durch das zweite Langloch wieder unter die Stoma- Schutzkappe geführt zu werden.

In einer alternativen Ausführungsform sind die Führungen als Bügel ausgebildet, die auf der Vorderseite der Stoma-Schutzkappe angeordnet sind und die ein Unterschieben der Kleidung unter die Bügel erlauben.

In einer weiteren Ausführungsform ist die Stoma- Schutzkappe grundsätzlich nach unten geöffnet, wobei hierfür die Wölbung nach unten eine Öffnung bildet, so dass der Beutel nach unten ausreichend Freiraum hat.

Durch diesen Ansatz erreicht man gewisse Vorteile. Diese Vorteile umfassen:
1.) Es wird keine Bauchbinde (Bruchband)benötigt, da die Stoma- Schutzkappe auch auf bzw. an den üblichen Stomagürtel oder an der Unterhose mit seinen seitlichen Führungsschlitzen befestigt werden kann. Auch kann ein eigens dafür gefertigter Gürtel verwendet werden.
2.) Durch die Größe der Stoma- Schutzkappe wird der Stomabeutel nicht zusammen gedrückt und das Stoma kann den Stuhl ungehindert unter die Stoma-Schutzkappe in den Beutel austreten lassen.
3.) Die Stoma- Schutzkappe kann mit den seitlichen Führungsschlitzen auch unter den Hosenbund oder Rockbund geschoben werden, so dass die Stoma-Schutzkappe über der Unterwäsche oder auch dem eingesteckten Hemd bzw. Bluse liegt. Somit wird verhindert, dass von der Kleidung Druck auf das Stoma ausgeübt wird. Auch tritt beim Laufen kein Scheuern am Stoma auf.

Im Folgenden werden die Figuren kurz beschrieben.
Fig. 1a-1b zeigen eine Stoma- Schutzkappe, die am Körper durch einen Gürtel gehalten wird.
Fig. 2a-2c zeigen die Stoma- Schutzkappe mit langlochförmigen Führungen von oben, von vorne und von unten.
Fig. 3a-3c zeigen die Stoma- Schutzkappe mit Bügel von oben, von der Seite und von vorne.
Fig. 3d-e zeigen die Stoma-Schutzkappe mit Bügel, wobei der Bügel an einer alternativen Position ausgebildet ist, so dass er an handelsüblichen Stomagürteln befestigt werden kann.
Fig. 4 zeigt eine Befestigung eines Stomagürtels an der Schutzkappe.
Fig. 5a zeigt die Schutzkappe mit Magneten und Langlöchern für die Befestigung eines konventionellen Stomagürtels
Fig. 5b zeigt die Magneten an einer Stomaschließhilfe, wie sie in einer parallelen Anmeldung veröffentlicht wurde.

### Detaillierte Beschreibung der Figuren:

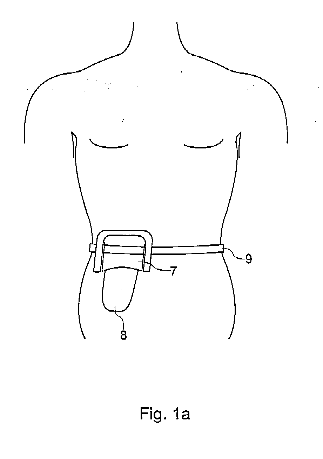

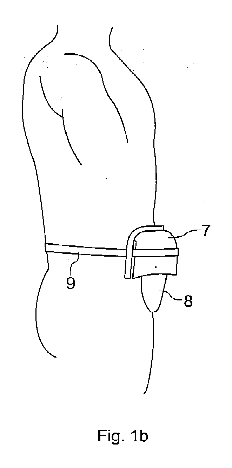

Die Figuren 1a bis 1b zeigen eine Stoma- Schutzkappe 7 am Körper einer Person. Hierbei befindet sich unterhalb der Stoma-Schutzkappe ein Auffangbeutel, der mit dem Stoma verbunden ist. Die Stoma- Schutzkappe wird an einen Gürtel oder einen Bund 9 geklemmt.

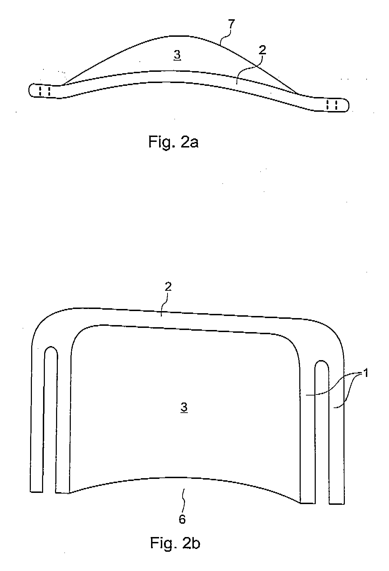

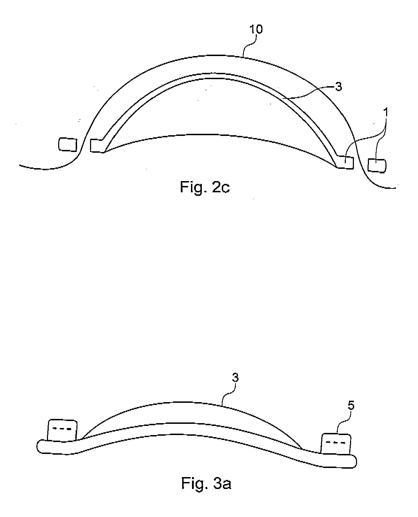

Die Figuren 2a bis 2c zeigen eine bevorzugte Ausführung der Stoma- Schutzkappe aus unterschiedlichen Perspektiven. Es ist deutlich zu erkennen, dass die Stoma- Schutzkappe eine Auswölbung beziehungsweise eine ausgewölbte Kappe 3 aufweist, die es ermöglicht, dass der Druck, der durch die Kleidung auf den Stoma und den Auffangbeutel erzeugt wird, abgeleitet wird und seitlich auf den Körper übertragen wird. Damit die Stoma-Schutzkappe Halt findet, sind seitliche Führungen 1 vorgesehen, durch die sich die Kleidung bzw. der Gürtel oder der Hosenbund teilweise erstreckt. Der Randbereich der Stoma- Schutzkappe ist verstärkt ausgebildet, wobei die gewölbte Kappe vorzugsweise dünner und elastischer ausgebildet ist und nach unten offen (Öffnung 6) ist, damit der Auffangbeutel dort austreten kann.
Die Führungen sind als Langloch- Schlitze ausgebildet, in die der Hosenbund bzw. der Gürtel eingefädelt werden kann. So tritt z. B. der Gürtel 10 anfänglich unter die Stoma- Schutzkappe, um dann durch den Schlitz nach außen bzw. nach vorne zu treten, um dann über die Kappe geleitet zu werden, damit er dann erneut durch die Führung kurzzeitig unter die Kappe tritt.

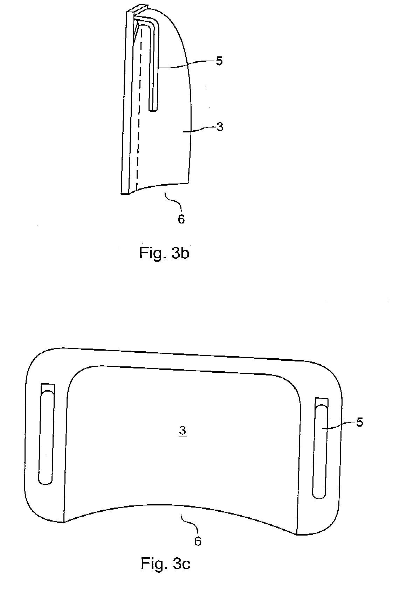

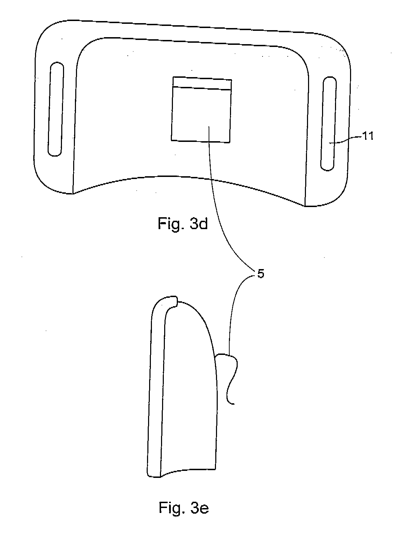

Die Figuren 3a bis 3c zeigen ein ähnliches Konzept, bei denen jedoch anstatt der Schlitze die Führungen als Laschen 5 ausgebildet sind, die im oberen Bereich an der Stoma-Schutzkappe befestigt sind und deren unterer Bereich freiliegend ist, so dass die Kleidung unter diese Lasche geklemmt werden kann. Die Lasche kann einstückig aus der Platte geformt sein oder mit dieser nachträglich verbunden worden sein z. B. durch Nieten, Kleben oder Schweißen.

Die Figuren 3d bis 3e zeigen die Stoma-Schutzkappe mit Bügel, wobei der Bügel an einer alternativen Position ausgebildet ist, so dass er an handelsüblichen Stomagürteln befestigt werden kann. Der Bügel ist hierbei mittig ausgebildet. Auch sind die Langlöcher 11 kürzer ausgebildet und beidseitig geschlossen, so dass der handelsüblichen Stomgürtel durch diesen geführt werden kann.

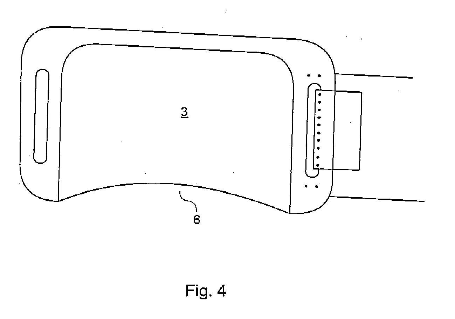

Die Fig. 4 zeigt eine Befestigung eines Stomagürtels an der Schutzkappe. Hierbei wird der Stomagürtel durch das Langloch geführt und mit einer Schnalle verbunden. Das Langloch ist dabei zu beiden Seiten begrenzt. Ein Herausrutschen wird somit vermieden.

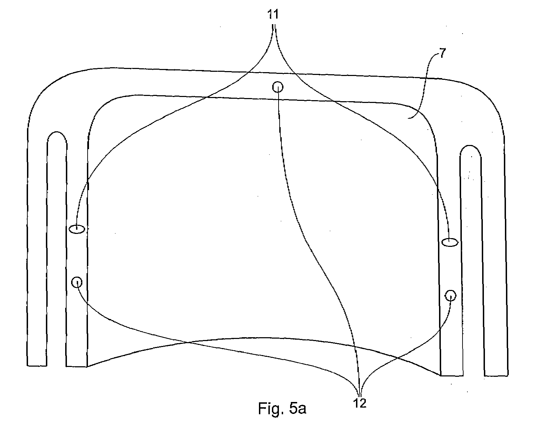

Fig. 5a zeigt die Schutzkappe mit Magneten 12 und Langlöchern 11 für die Befestigung eines konventionellen Stomagürtels. Hierbei sind entsprechende Mangneten sowohl auf dem Gürtel als auch auf der Schutzkappe angeordnet und erlauben somit einen sicheren Halt. Auch ist es denkbar, dass Klettverschlüsse verwendet werden.

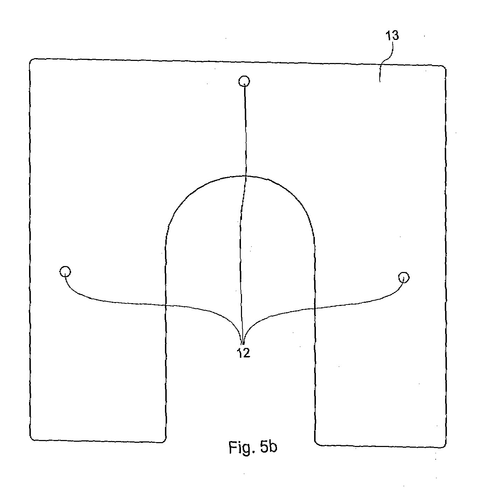

Die Fig. 5b zeigt die Magneten an einer Stomaschließhilfe 13, wie sie in einer parallelen Anmeldung beschrieben wurde, die am gleichen Tag eingereicht wurde. Der Inhalt dieser Anmeldung ist Teil der vorliegenden Anmeldung. Aufgrund der gleichen Anordnung der Magnete können diese für unterschiedliche Einsatzgebiete Haltefunktionen übernehmen.

Es wird darauf hingewiesen, dass die Stomaschutzkappe natürlich auch aus Pappe oder einem ähnlichen Zellstoffprodukt gefertigt sein kann und ein Wegwerfartikel darstellt, der nur einmal zusammen mit dem Auffangbeutel verwendet wird.

### Bezugszeichenliste

- 1: Führungen
- 2: Oberer Rand
- 3: Gewölbte Kappe
- 4: Platte
- 5: Klemmbügel
- 6: Öffnung
- 7: Stoma- Schutzkappe
- 8: Auffangbeutel
- 9: Gürtel
- 10: Gürtel bzw. Bund
- 11: Langloch für Stoma-Gürtel
- 12: Magneten
- 13: Stomaschließhilfe

## Patentansprüche

1. Stoma-Schutzkappe mit einer um ein Stoma eines Benutzers anordenbare Platte, die eine Wölbung aufweist, die im Gebrauchszustand sich mit einem Abstand über dem Stoma befindet, wobei die Platte einen Haltebereich zur Befestigung an der Kleidung des Benutzers aufweist, **dadurch gekennzeichnet, dass** am Haltebereich eine schlitzartige Führung ausgebildet ist, wodurch die Stoma-Schutzkappe auf den Gürtel oder den Hosenbund des Benutzers aufsteckbar ist, so dass ein Teil der Führung und der Bereich der Wölbung der Platte sich im aufgesteckten Zustand zwischen Hosenbund oder Gürtel und Bauchdecke des Benutzers befindet, während der übrige Teil der Führung sich auf der dem Benutzer abgewandten Seite des Gürtels oder des Hosenbundes befindet.

2. Stoma-Schutzkappe nach Anspruch 1, **dadurch gekennzeichnet, dass** die Führung durch jeweils an einer Seite mit der Platte verundene, mit einem Abstand parallel zur Oberfläche der Platte verlaufende Bügel gebildet ist.

3. Stoma-Schutzkappe nach einem der Ansprüche 1 bis 3, **dadurch gekennzeichnet, dass** die Führung durch Schlitze in der Platte in einem Bereich außerhalb der Wölbung gebildet ist.

4. Stoma-Schutzkappe nach einem der Ansprüche 1 bis 3, **dadurch gekennzeichnet, dass** die Wölbung im Gebrauchszustand eine Öffnung nach unten zum Durchtritt eines Auffangbeitels frei lässt.
